# EUROPEAN PATENT APPLICATION

(11) **EP 4 636 079 A1**
(43) Date of publication of application: **22.10.2025**
(21) Application number: 23903899.5
(22) Date of filing: 08.12.2023
(51) Int. Cl.: C12N 5/079

(54) **METHOD FOR INDUCING DIFFERENTIATION OF STEM CELLS INTO CORNEAL ENDOTHELIAL CELLS AND USE THEREOF**

(30) Priority: 13.12.2022 KR 20220173474; 24.02.2023 KR 20230025121
(71) Applicant: The Asan Foundation, Seoul 05505 (KR); University Of Ulsan Foundation For Industry Cooperation, Ulsan 44610 (KR)
(72) Inventor: LEE, Hun, Seoul 05505 (KR); YE, Eun Ah, Seoul 05505 (KR)
(74) Representative: Bryn Aarflot AS
(86) International application number: PCT/KR2023/020200
(87) International publication number: WO 2024/128696

(57) **Abstract**

The present invention relates to a method of inducing differentiation of stem cells into corneal endothelial cells and use thereof. The method according to an aspect may increase, by using a composition for differentiation, the efficiency of differentiating stem cells into corneal endothelial cells. The differentiated corneal endothelial cells exhibit specific characteristics of corneal endothelial cells, and thus can be widely used as an alternative cell source for transplantation of corneal endothelial cells.

Additionally, since FBS is not contained, stability in clinical trials may be improved.

## Description

### Technical Field

The present invention relates to a method of inducing differentiation of stem cells into corneal endothelial cells and use thereof.

### Background Art

The cornea is the outermost structure of the eye, and is one of main organs that refract light. The cornea consists of a total of six layers: corneal epithelium, Bowman's layer, corneal stroma, Dua's layer, Descemet's membrane, and corneal endothelium.

The corneal endothelium has a single-layer structure of hexagonal cells on the posterior surface of the cornea, and contains physiological ion pumps. At birth, the average density of human corneal endothelial cells (CECs) is known to be about 5000 cells/mm². However, due to the limited mitotic potential, the total number of cells decreases with age.

When the CECs are damaged, the cells repair through cell expansion and migration rather than through mitosis, and in this regard, the cornea swells to several times its normal thickness, becomes opaque, and loses its function. Here, the damage is permanent.

Currently, the established treatment for CEC damage is keratoplasty. Such damage may be repaired by transplantation of donor tissue through penetrating keratoplasty (PK) or lamellar keratoplasty.

However, corneal donors are in short supply, and to overcome this, there is a need to develop compositions and methods that can efficiently induce differentiation of stem cells, such as induced pluripotent stem cells (iPSCs) and embryonic stem cells, into CECs.

### Description of Embodiments

### Technical Problem

One aspect is to provide a method of differentiating stem cells into corneal endothelial cells, the method including culturing stem cells in a culture vessel coated with a modified extracellular matrix (ECM) in the presence of a composition containing a Rho-associated protein kinase (ROCK) inhibitor.

Another aspect is to provide a kit for inducing differentiation of stem cells into corneal endothelial cells, the kit including: a composition containing a ROCK inhibitor; and a culture vessel coated with a modified ECM.

### Solution to Problem

One aspect provides a method of differentiating stem cells into corneal endothelial cells, the method including culturing stem cells in a culture vessel coated with a modified extracellular matrix (ECM) in the presence of a composition containing a Rho-associated protein kinase (ROCK) inhibitor.

In the present invention, the term "stem cells" may refer to totipotent cells that can differentiate into all types of cells or to pluripotent cells that can differentiate into several types of cells, and the stem cells are undifferentiated cells that can differentiate into cells of a specific tissue.

In an embodiment, the stem cells may be embryonic stem cells (ESCs), adult stem cells, or induced pluripotent stem cells (iPSCs).

The ESCs may be derived from the inner cell mass of a blastocyst embryo just before implantation of a fertilized egg in the uterus of a mother and then cultured in vitro, and the adult stem cells are undifferentiated cells that are present in an extremely small amount in each tissue of the body and may be cells replacing dead cells or damaged tissue. The iPSCs may be cells that have been induced to become pluripotent, such as ESCs, by injecting genes associated with cell differentiation into differentiated somatic cells and returning them to a cell stage prior to differentiation.

In the present specification, the term "Rho-associated protein kinase (ROCK) inhibitor" may refer to a material inhibiting ROCK, and the ROCK inhibitor may be used interchangeably with a ROCK suppressor.

In an embodiment, the ROCK inhibitor may include at least one selected from the group consisting of fasudil, N-(4-pyridinyl)-4β-[(R)-1-aminoethyl]cyclohexane-1αcarboamide (Y-27632), (2S)-2-methyl-1-[(4-methyl-5-isoquinolinyl)sulfonyl]hexahydro-1H-1,4-diazepine (H-1152), 4β-[(1R)-1-aminoethyl]-N-(4-pyridyl)benzene-1zencarboamide (Wf-536), N-(1H-pyrrolo[2,3-b]pyridin-4-yl)-4PER(R)-1-aminoethyl]cyclohexane-1 rohecarboamide (Y-30141), N-(3-{[2-(4-amino-1 ,2,5-oxadiazol-3-yl)-1-ethyl-1 H-imidazo[4,5-c]pyridin-6-yl]oxy}phenyl)-4-{[2-(4-morpholinyl)ethyl]-oxy}benzamide (GSK269962A), and N-(6-fluoro-1H-indazol-5-yl)-6-methyl-2-oxo-4-[4-(trifluoromethyl)phenyl]-3,4-dihydro-1H-pyridin-5-carboxamide (GSK429286A).

In the present specification, the term "extracellular matrix (ECM)" may refer to a complex assembly of biopolymers that fill a space within a tissue or an extracellular space, and may include molecules synthesized by cells and secreted and accumulated extracellularly. The term "ECM analogue" is a mixture having a composition similar to the ECM consisting of a three-dimensional network, and may refer to a mixture of artificial or natural proteins that has the same or similar biological functions as a natural ECM.

In an embodiment, the ECM may contain at least one selected from the group consisting of vitronectin, collagen, fibronectin, laminin, gelatin, matrigel, hyaluronic acid, polylysine, and heparan sulfate proteoglycan.

In the present specification, the term "culture vessel" may refer to a plate for cell culture or the like, typically used for cell culture or biochemical experiments, but may include, without limitation, a dish, a flask, a multi-well plate, and the like.

In the present specification, the term "coating" may refer to forming a new layer with a constant thickness by forming a film of a specific material on a target surface. The target surface may be coated with a coating material via a variety of chemical bonds, such as an ionic bond, a covalent bond, a hydrogen bond, and the like. When the surface of the culture vessel is modified with the ECM or the like, the ECM may form a sealed layer completely surrounding the surface of the culture vessel, or may form a partially sealed layer.

For use as a medium suitable for the culturing, any medium typically used in culture and/or differentiation of stem cells may be used without limitation. The medium may contain various nutrient sources required to maintain proliferation of cells or components required to induce differentiation, and examples of the nutrient sources may include: a carbon source such as glycerol, glucose, fructose, sucrose, lactose, honey, starch, dextrin, etc.; a hydrocarbon such as fatty acid, fat, lectin, alcohol, etc.; a nitrogen source such as ammonium sulfate, ammonium nitrate, ammonium chloride, urea, sodium nitrate, etc.; an inorganic salt such as a table salt, a potassium salt, a phosphate, a magnesium salt, a calcium salt, an iron salt, a manganese salt, etc.; and potassium phosphate monobasic, potassium phosphate dibasic, magnesium sulfate, sodium chloride, ferrous sulfate, sodium molybdate, sodium tungstate, manganese sulfate, various vitamins, amino acids, etc.

The culturing may be performed at a temperature of 30 °C to 40 °C, for example, under temperature conditions of 35 °C to 38 °C, and preferably, under a temperature condition of 37 °C.

In an embodiment, the method of differentiating may include: differentiating the stem cells into neural crest cells (NCCs) by culturing the stem cells in a culture vessel to which a first medium is added; and differentiating the NCCs into corneal endothelial cells (CECs) by culturing the NCCs in a culture vessel to which a second medium is added.

In the present specification, the term "neural crest cells (NCCs)" may refer to cells generated at the interface between the neural tube and the epithelial ectoderm during formation of the central nervous system in the embryonic development and then migrating to various regions of the body through the epithelial-mesenchymal transition (EMT)

In the present specification, the term "corneal endothelial cells (CECs)" may refer to cells constituting the corneal endothelium.

The first medium may contain the ROCK inhibitor, and may additionally contain components suitable for inducing the differentiation of the stem cells into NCCs. For example, the first medium may be Essential 6 medium, and medium components may include fasudil, LDN193189, BGJ398, CHIR99021, FGF2, SAG, BGJ398, BMP4, or a combination thereof.

The differentiating of the stem cells into NCCs may include culturing the stem cells in the culture vessel to which the first medium is added for 1 to 10 days, for example, 1 to 9 days, 1 to 8 days, 1 to 7 days, 1 to 6 days, 1 to 5 days, 1 to 4 days, 1 to 3 days, 1 to 2 days, 2 to 10 days, 2 to 9 days, 2 to 8 days, 2 to 7 days, 2 to 6 days, 2 to 5 days, 2 to 4 days, 2 to 3 days, 3 to 10 days, 3 to 9 days, 3 to 8 days, 3 to 7 days, 3 to 6 days, 3 to 5 days, 3 to 4 days, 4 to 10 days, 4 to 9 days, 4 to 8 days, 4 to 7 days, 4 to 6 days, 4 to 5 days, 5 to 10 days, 5 to 9 days, 5 to 8 days, 5 to 7 days, 5 to 6 days, 6 to 10 days, 6 to 9 days, 6 to 8 days, 6 to 7 days, 7 to 10 days, 7 to 9 days, 7 to 8 days, 8 to 10 days, 8 to 9 days, or 9 to 10 days.

The second medium may contain the ROCK inhibitor, and may additionally contain components suitable for inducing differentiation of the NCCs into CECs. For example, the second medium may be Human Endothelial-SFM, and may include, as medium components, polyvinyl alcohol, insulin-transferrin-selenium, calcium chloride, 2-phosphate ascorbic acid, SB431542, fasudil, H-1152, Y27632, or a combination thereof.

The differentiating of the NCCs into CECs may include culturing the NCCs in the culture vessel to which the second medium is added for 2 to 15 days, for example, 2 to 14 days, 2 to 12 days, 2 to 10 days, 2 to 8 days, 2 to 6 days, 2 to 4 days, 3 to 15 days, 3 to 14 days, 3 to 12 days, 3 to 10 days, 3 to 8 days, 3 to 6 days, 3 to 4 days, 4 to 15 days, 4 to 14 days, 4 to 12 days, 4 to 10 days, 4 to 8 days, 4 to 6 days, 6 to 15 days, 6 to 14 days, 6 to 12 days, 6 to 10 days, 6 to 8 days, 7 to 15 days, 7 to 14 days, 7 to 12 days, 7 to 10 days, 7 to 8 days, 8 to 15 days, 8 to 14 days, 8 to 12 days, 8 to 10 days, 10 to 15 days, 10 to 14 days, 10 to 12 days, or 10 to 15 days.

In an embodiment, the composition may contain polyvinyl alcohol (PVA).

The composition may be fetal bovine serum (FBS)-free.

In the present specification, the expression " substantially does not comprise" or" does not comprise" may refer that the FBS is included to a degree that does not affect the properties, activities, or pharmacological activities of the composition, or is not included at all. For example, the FBS may be included in an amount of 0.02 wt% or less, 0.01 wt% or less, 0.005 wt% or less, or 0.001 wt% or less, based on the total weight of the composition, or may not be included at all.

When the composition is FBS-free, but includes PVA, stability in clinical trials may be improved.

In the present specification, the term "differentiation" may refer to a phenomenon in which cells become more specialized in structure or function while the cells divide, multiply, and grow, and that is, may refer that the cells, tissues, and the like of a living organism change in form or function to perform the tasks for which they are given. In addition, in the present specification, the term "differentiation potency" refers to the ability to differentiate, and the term "cells having differentiation potency" may refer to cells having the ability to differentiate into tissues or cells that constitute a living organism.

In the present specification, the expression "induction of differentiation of the stem cells into CECs" may include not only induction of complete differentiation of the stem cells including iPSCs into CECs, but also induction of partial differentiation of the stem cells into cells in which a CEC-specific marker is expressed.

In an embodiment, the composition may decrease expression of a pluripotent stem cell-specific marker, or may increase expression of a CEC-specific marker.

The pluripotent stem cell-specific marker may include at least one selected from the group consisting of Octamer-binding transcription factor 4 (OCT4), SRY-Box Transcription Factor 2 (SOX2), and NANOG.

The CEC-specific marker may include at least one selected from Zonula occludens-1 (ZO-1), neuronal cell adhesion molecule (NCAM), Solute Carrier Family 4 Member 11 (SLC4A11), N-Cadherin, Collagen Type VIII Alpha 1 Chain (COL8A1), Aquaporin 1 (AQP1), and ATPase Na+/K+ Transporting Subunit Alpha 1 (ATP1A1).

The composition may induce the differentiation of the stem cells into CECs by degrading properties of pluripotent stem cells and increasing unique properties of CECs, during a process of inducing the differentiation of the stem cells into CECs.

Another aspect provides a kit for inducing differentiation of stem cells into CECs, the kit including: a composition including a ROCK inhibitor; and a culture vessel coated with a modified ECM.

The ROCK inhibitor, the composition, the ECM, the culture vessel, the stem cells, and the CECs are the same as described above.

### Advantageous Effects of Disclosure

According to the method of an aspect, use of the composition for differentiation may increase efficiency of differentiating stem cells into corneal endothelial cells, and since the differentiated corneal endothelial cells exhibit specific characteristics of corneal endothelial cells, these cells may be widely used as an alternative cell source for corneal endothelial cell transplantation.

Additionally, since the composition is FBS-free, stability in clinical trials may be improved.

### Brief Description of Drawings

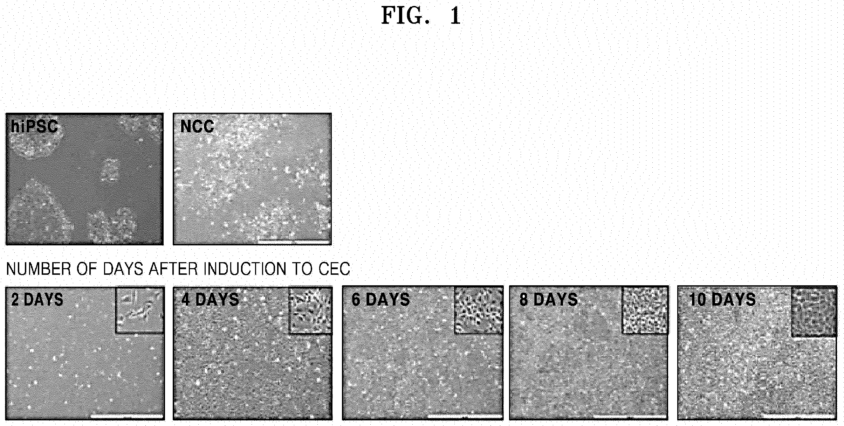

FIG. 1 shows microscopic images of hiPSCs, hiPSC-derived NCCs, and cells on Days 2, 4, 6, 8, and 10 of NCC to CEC induction, to confirm changes in morphology during a process of differentiating hiPSCs into CECs via NCCs according to an aspect.

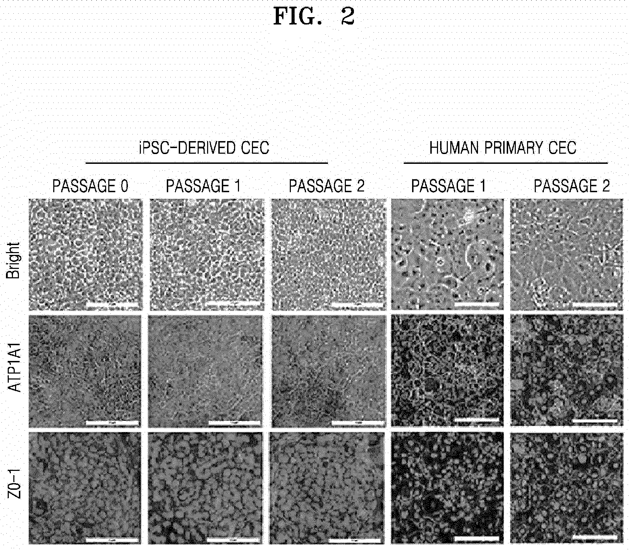

FIG. 2 shows images determining whether CEC-specific markers ATP1A1 and ZO-1 are expressed in CECs differentiated from iPSCs according to an aspect.

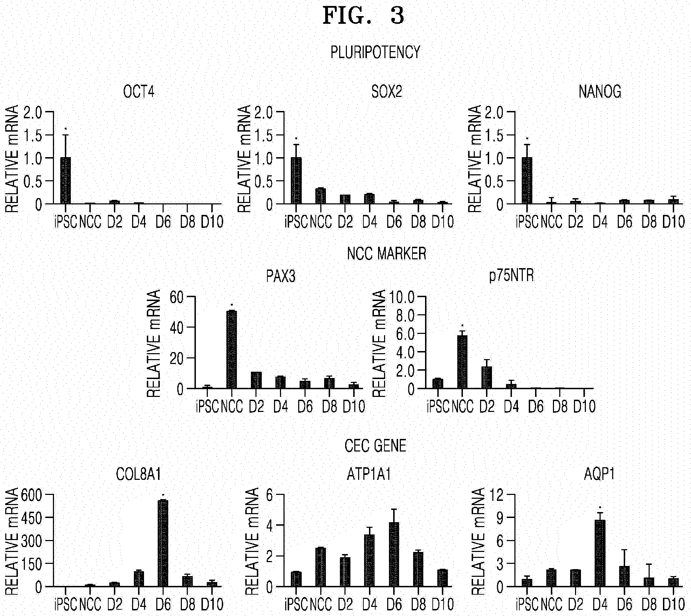

FIG. 3 shows graphs showing changes in expression levels of each of pluripotent stem cell-specific markers (OCT4, SOX2, and NANOG), NCC-specific markers (p75NTR and PAX3), and CEC-specific markers (ATP1A1, COL8A1, and AQP1), during a process of differentiating iPSCs into CECs via NCCs according to an aspect, wherein iPSC indicates induced pluripotent stem cells, NCC indicates iPSC-induced neural crest cells, D2 indicates cells on Day 2 of NCC to CEC induction, D4 indicates cells on Day 4 of NCC to CEC induction, D6 indicates cells on Day 6 of NCC to CEC induction, D8 indicates cells on Day 8 of NCC to CEC induction, and D10 indicates cells on Day 10 of NCC to CEC induction.

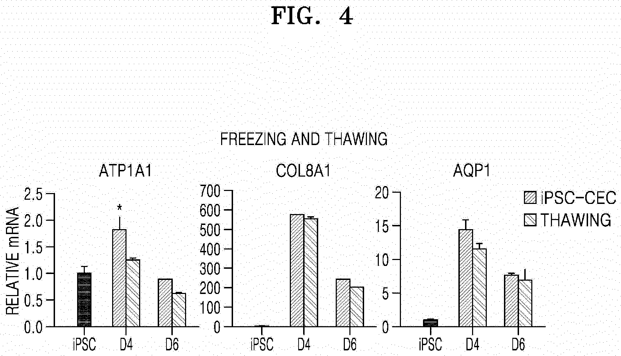

FIG. 4 shows graphs determining whether CEC-specific markers (ATP1A1, COL8A1, and AQP1) are expressed before and after freezing and thawing of CECs, wherein the CECs are differentiated according to an aspect and undergo freezing and thawing; iPSC indicates induced pluripotent stem cells, D4 indicates cells on Day 4 of induction of iPSC-derived NCC to CEC, and D6 indicates cells on Day 6 of induction of iPSC-induced NCC to CEC.

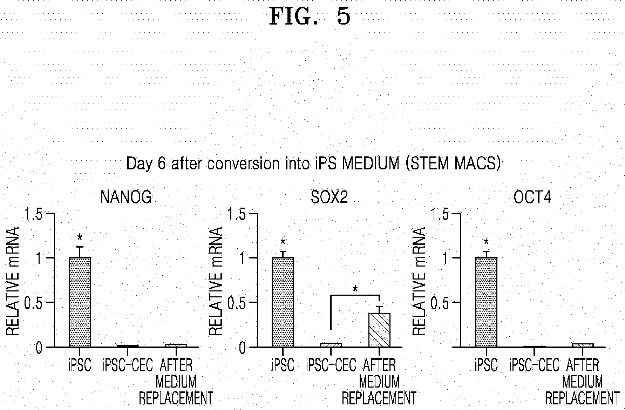

FIG. 5 shows graphs measuring expression of pluripotent markers at 6 days after culturing CECs differentiated from iPSCs according to an aspect in iPS-Brew XF medium.

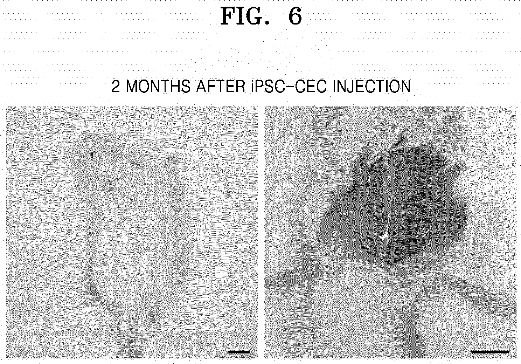

FIG. 6 shows images of a mouse injected with CECs differentiated from iPSCs according to an aspect and examined 2 months later for the formation of teratomas in the mouse.

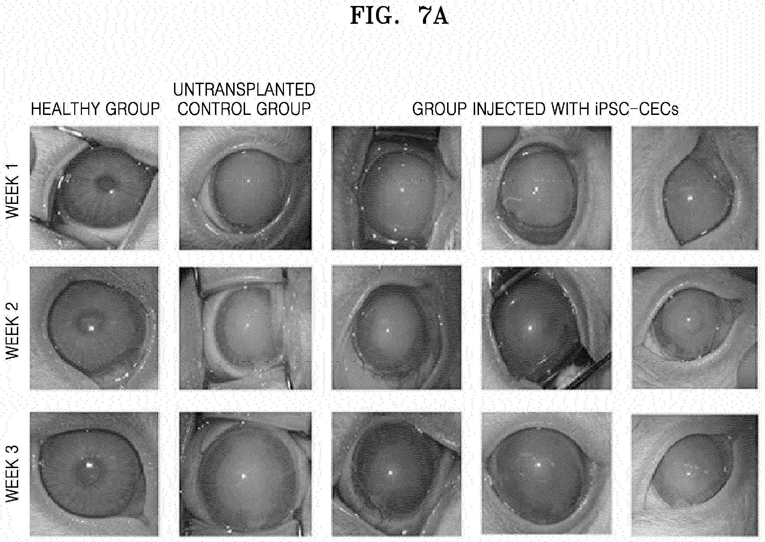

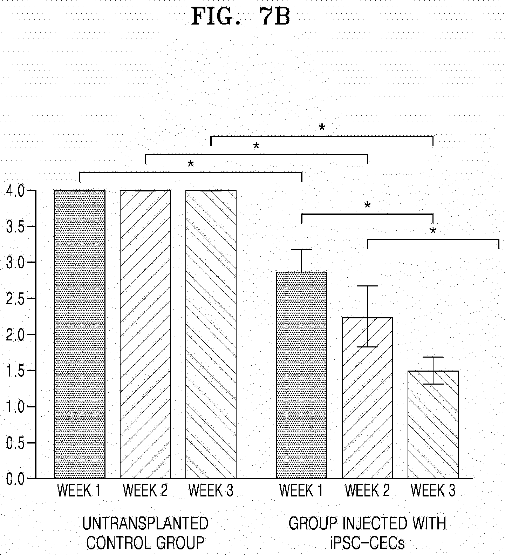

FIG. 7A shows images determining whether a clouding phenomenon is improved after transplantation of CECs differentiated from iPSCs into a rabbit model of corneal endothelial dysfunction (CED), compared to the cornea of an untransplanted CEB model, and FIG. 7B is a graph showing changes in corneal opacity over time in a group transplanted with CECs differentiated from iPSCs and a control group not transplanted with the CECs.

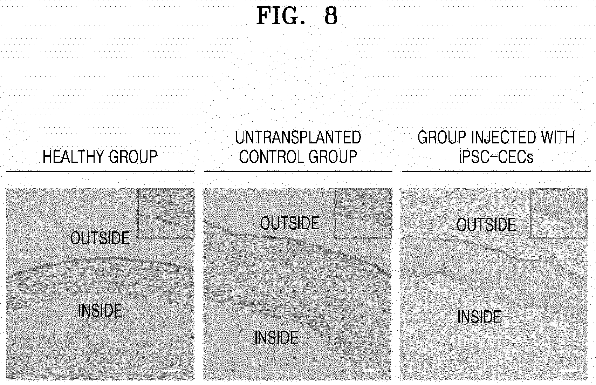

FIG. 8 shows images confirming changes in a corneal thickness by H&E staining after transplanting CECs differentiated from iPSCs into a CED rabbit, compared to the cornea of an untransplanted CED model; Scale bars: 200 µm.

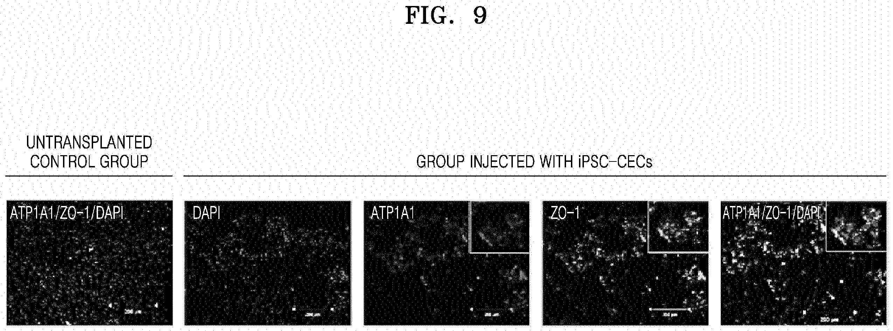

FIG. 9 shows images analyzing corneal tissue in a group transplanted with CECs at 3 weeks after the transplantation, compared to corneal tissue of a group not transplanted with the CECs.

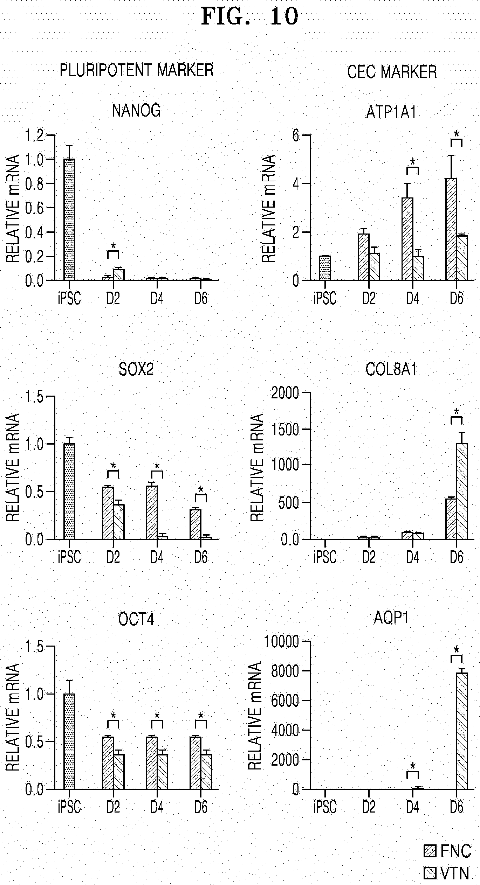

FIG. 10 shows graphs measuring and comparing changes in expression levels of PSC-specific markers (NANOG, SOX2, and OCT4) and CEC-specific markers(ATP1A1, COL8A1, and AQP1) during a process of differentiating stem cells into CECs via NCCs according to an aspect; iPSC indicates induced pluripotent stem cells, D2 indicates cells on Day 2 of induction of iPSC-derived **NCCs to CECs,** D4 indicates cells on Day 4 of induction of iPSC-derived NCCs to CECs, and D6 indicates cells on Day 6 of induction of iPSC-derived NCCs to CECs.

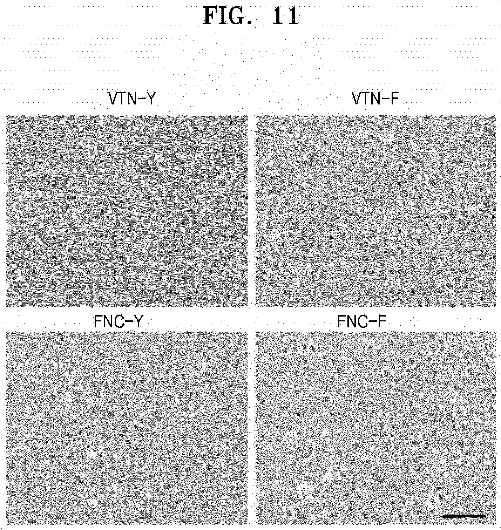

FIG. 11 is an image confirming morphology of each cell among cells induced to differentiate from iPSCs to CECs through different combinations of a coating material and a ROCK inhibitor: VTN-Y indicates a combination of vitronectin and Y27632; VTN-F indicates a combination of vitronectin and fasudil; FNC-Y indicates a combination of FNC mixture and Y27632; and FNC-F indicates a combination of FNC mixture and fasudil.

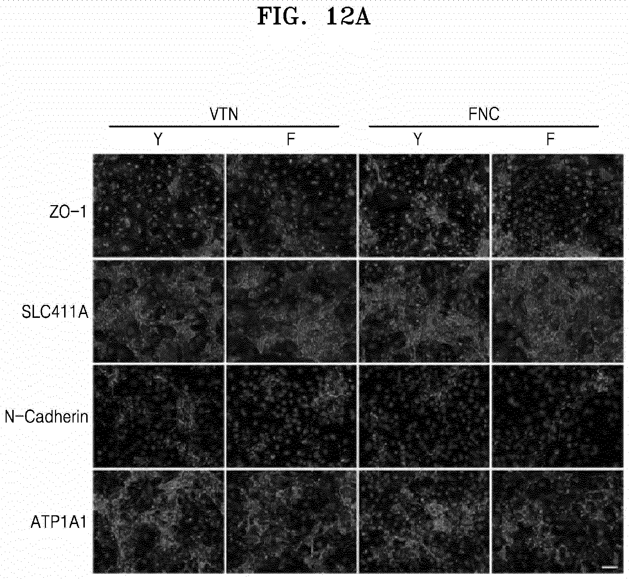

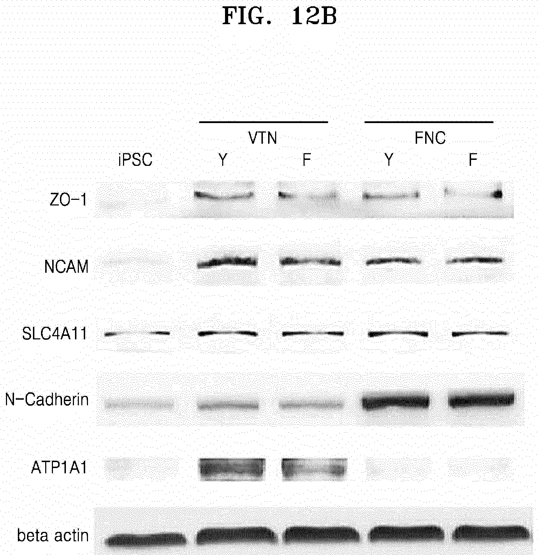

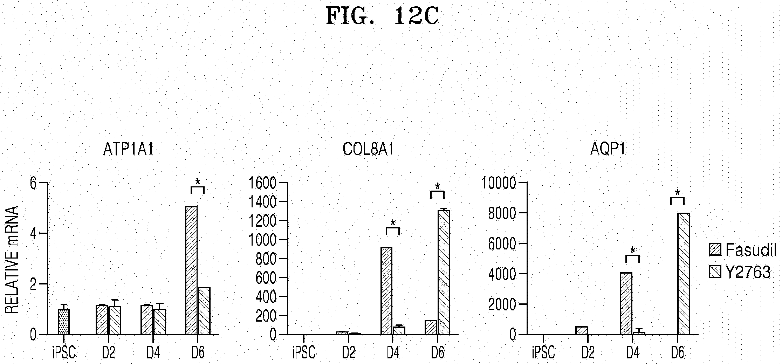

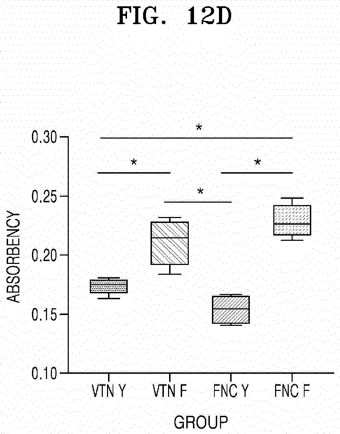

FIG. 12A shows images measuring expression levels of CEC-specific markers ZO-1, SLC4A11, N-Cadherin, and ATP1A1 in response to a combination of a coating material and a ROCK inhibitor during a process of iPSC to CEC induction according to an aspect; FIG. 12B shows images measuring expression levels of CEC-specific markers ZO-1, NCAM, SLC4A11, N-Cadherin, and ATP1A1 in response to a combination of a coating material and a ROCK inhibitor during a process of iPSC to CEC induction according to an aspect; FIG. 12C shows graphs measuring expression levels of CEC-specific markers ATP1A1, COL8A1, and AQP1 in response to a combination of a coating material and a ROCK inhibitor during a process of iPSC to CEC induction according to an aspect, wherein iPSC indicates induced pluripotent stem cells, D2 indicates cells on Day 2 of induction of iPSC-derived NCCs to CECs, D4 indicates cells on Day 4 of induction of iPSC-derived NCCs to CECs, and D6 indicates cells on Day 6 of induction of iPSC-derived NCCs to CECs; and FIG. 12D is a graph showing cell viability and cell proliferation levels in response to a combination of a coating material and a ROCK inhibitor during a process of iPSC to CEC induction according to an aspect, wherein VTN-Y indicates a combination of vitronectin and Y27632, VTN-F indicates a combination of vitronectin and fasudil, FNC-Y indicates a combination of FNC mixture and Y27632, and FNC-F indicates a combination of FNC mixture and fasudil.

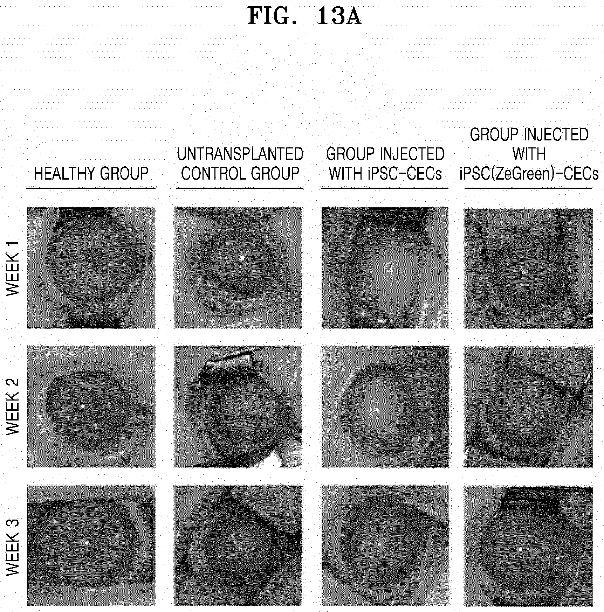

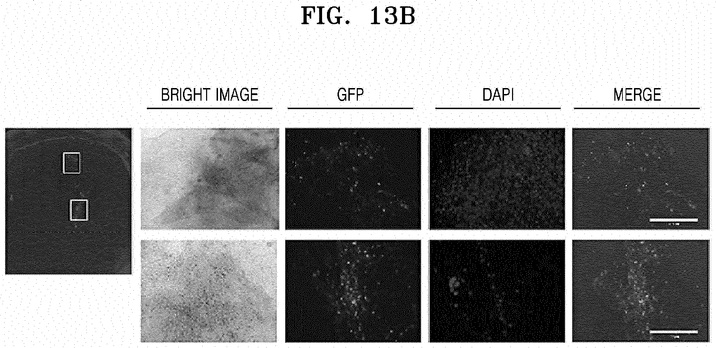

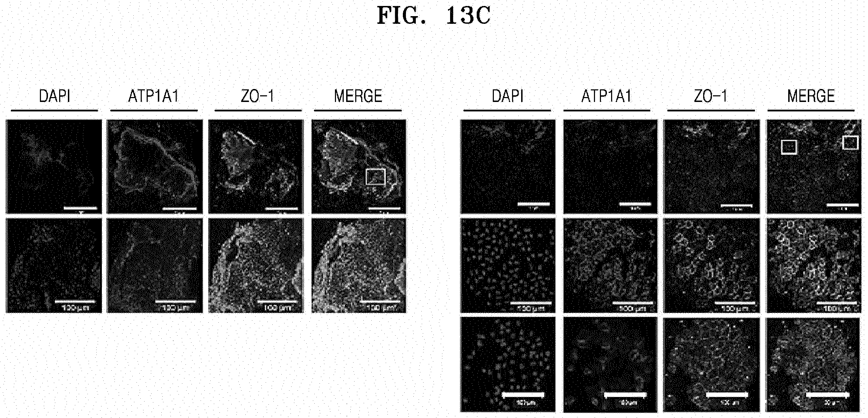

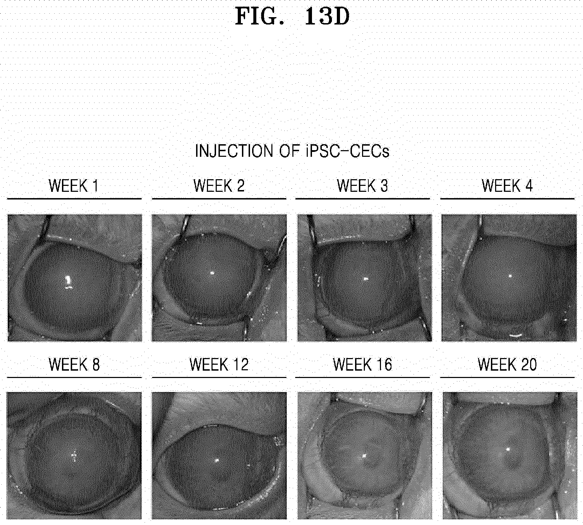

FIG. 13A shows images confirming changes in corneal opacity in a CED rabbit model after transplanting CECs induced in response to a combination of vitronectin and fasudil (i.e., iPSC-CECs(VTN with fasudil)) to the cornea of the CED rabbit model; FIG. 13B shows images confirming the CECs expressing a green fluorescent protein observed in the cornea transplanted with iPSC(ZsGreen)-derived CECs (i.e., iPSC(ZsGreen)-CECs(VTN with fasudil)); FIG. 13C shows images confirming positive cells expressing human CEC-specific markers ZO-1 and ATP1A1 in corneal tissue transplanted with the iPSC-CECs(VTN with fasudil); and FIG. 13D shows images confirming changes in corneal opacity observed for 20 weeks after transplanting the iPSC-CECs(VTN with fasudil)) into the cornea of the CED rabbit model.

### Mode for Invention

Hereinafter, the present invention will be described in detail with reference to Examples below. However, these Examples are for illustrative purposes only, and the scope of the present invention is not intended to be limited by these Examples.

### Reference Example 1. Quantitative RT-PCR

Total RNAs from iPSCs, iPSC-derived NCCs, and iPSC-derived CECs were extracted using TRIzol reagent (Invitrogen), and complementary DNA was synthesized using a kit (Superscript III; Invitrogen). Quantitative RT-PCR (qRT-PCR) was performed on the Step One ABI Real-Time PCR System (Applied Biosystems) using Power SYBR Green PCR Master Mix(Applied Biosystems, CA, USA).

The expression levels of PSC-specific markers (NANOG, OCT4, and SOX2), NCC-specific markers (p75NTR and PAX3), and CEC-related markers(ATP1A1, COL8A1, and AQP1) were measured at different time points during differentiation of iPSCs to CECs. Here, the mRNA level was normalized by the GAPDH.

### Reference Example 2. Immunocytochemical staining

The CECs were fixed with 4 % paraformaldehyde overnight, washed with PBST buffer, permeabilized in 0.5 % Triton X-100, and blocked in PBST containing 1 % BSA (by Sigma-Aldrich). Samples were cultured with primary antibodies, such as anti-human anti-ZO-1 (1:250; Life Technologies) antibody and anti-Na+/K+ATPase α1 antibody (1:200, Santa Cruz Biotechnology, Dallas, TX, USA), at 4 °C overnight, followed by goat anti-mouse IgG Alexa Fluor 488-conjugated and donkey anti-rabbit IgG Alexa Fluor 647-conjugated secondary antibodies (1:500; Abcam, Cambridge, UK). The nuclei were counterstained with 4',6-diamidino-2-phenylindole (DAPI; Sigma-Aldrich) for 10 minutes, and fluorescence signals were detected by a laser scanning confocal microscope (Olympus, Tokyo, Japan) or a fluorescent microscope (EVOS, Life Technologies).

### Reference Example 3. Western blot analysis

Cells differentiated into CECs were harvested and lysed in RIPA lysis buffer (EPIS BIO, South Korea) containing phosphatase and protease inhibitors (Sigma-Aldrich)according to the manufacturer's protocol. Cell lysates transferred to a PVDF membrane (Sigma-Aldrich) using Trans-Blot-Cell (Bio-Rad Laboratories Inc, Richmond, CA, USA) were separated on 8 % SDS-polyacrylamide gel (SDS-PAGE). Non-specific protein bonds were blocked with 3 % BSA (Sigma-Aldrich) in PBS/0.05 % Tween 20 (PBST). The membrane was incubated overnight with ZO-1 antibody(1:1,000) and β-actin antibody (1:1,000; Cell signaling technology, Danvers, MA, USA). The membrane was washed and incubated with horseradish peroxidase-conjugated anti-mouse secondary antibodies (GeneTex, Hsinchu City, Taiwan) at a dilution of 1:10,000. Detection was performed using Gel DocTM XR+(Bio-Rad Laboratories Inc) according to the manufacturer's instructions.

### Example 1. Differentiation of stem cells into CECs

Human induced pluripotent stem cells (hiPSCs) were provided by the Asan Stem Cell Center (Asan Institute for Life Sciences, Seoul). The hiPSCs were cultured in Stem MACS iPS-Brew XF medium on a vitronectin- modified plate under a serum-free condition.

For differentiation of the hiPSCs into NCCs, the hiPSCs were modified at low density on the surface of the vitronectin-coated plate. After the undifferentiated hiPSCs reached about 70 to 80 % cell density, a first medium was added to the plate to induce differentiation into NCCS. The first medium used was as follows: more specifically, first, the medium was replaced with NCC induction E6 medium (by Life Technologies) supplemented with 500 nM LDN193189(Selleckchem, Houston, TX, USA), 100 nM BGJ398(Selleckchem), and 10 µm fasudil(Adooq, Irvine, CA, USA), and cultured for 1 day. The medium was then replaced again with E6 medium supplemented with 1400 nM CHIR99021 (Peprotech), 100 ng/ml FGF2(Peprotech), 250 nM SAG(Selleckchem), and 10 µm fasudil, and cultured for 1 day. Next, the medium was then replaced again with E6 medium supplemented with 100 nM BGJ398, 20 ng/ml BMP4(Peprotech) and 10 µM fasudil, and cultured for 1 day. Next, for 2 to 4 days, the medium was replaced daily with E6 medium supplemented with 10 ng/ml FGF2 and 10 µM fasudil to induce differentiation into NCCs.

Also, for differentiation of the induced NCCs into CECs, the iPSC-derived NCCs were isolated with accutase (by Life Technologies). Then, the isolated cells were seeded onto a 35 mm-plate modified with FNC mixture (United States Biological, Salem, MA, USA) at low cell density(20 to 30 %). Then, for the next 10 days, the cells were cultured in human endothelial-SFM as a second medium supplemented with 0.1 % PVA (Sigma-Aldrich), 1 % insulin-transferrin-selenium (Life Technologies), 0.2 mg/mL CaCl₂(Sigma-Aldrich), 0.02 mg/mL 2-phosphate ascorbic acid, 1 µM SB431542 (Selleckchem), 2.5 µM ROCK inhibitor H-1152(Tocris, Abington, UK), and 10 µM Y27632 (Sigma-Aldrich) to induce differentiation into CECS. The cells differentiated into CECs were then passaged when reached a cell density of about 80 to 90 %.

### Example 2. Confirmation of characteristics of CECs differentiated from iPSCs

To determine whether the phenotype of CECs occurred during a process of differentiation from hiPSCs to CECs via NCCs, the morphology of cells on Days 2, 4, 6, 8, and 10 of NCC to CEC induction was observed using a microscope, and the results are shown in FIG. 1.

FIG. 1 shows microscopic images of hiPSCs, hiPSC-derived NCCs, and cells on Days 2, 4, 6, 8, and 10 of the NCC to CEC induction, to confirm changes in morphology during a process of differentiating hiPSCs into CECs via NCCs according to an aspect.

As shown in FIG. 1, it was confirmed that hexagonality, a representative characteristic of CECs, and tight cell junctions appeared from Day 6 of induction from NCCs to CECs.

In addition, the expression of CEC-specific markers ATP1A1 and ZO-1 was measured in iPSC-derived CECs by immunocytochemical staining using the method described in Reference Example 2, and the results are shown in FIG. 2.

FIG. 2 shows images determining whether CEC-specific markers ATP1A1 and ZO-1 are expressed in CECs differentiated from iPSCs according to an aspect.

As shown in FIG. 2, most cells according to an aspect expressed both ATP1A1 and ZO-1, and the expression of these markers was confirmed to be maintained even after two passages.

Additionally, to determine whether differentiation from hiPSCs to CECs via NCCs is successful by the method described in Reference Example 1, hiPSCs, the hiPSC-derived NCCs, and cells on Days 2, 4, 6, 8, and 10 of the NCC to CEC induction were each collected, and expression levels of representative PSC-specific markers (OCT4, SOX2, and NANOG), NCC-specific markers (p75NTR and PAX3), and CEC-specific markers (ATP1A1, COL8A1, and AQP1) were measured, and the results are shown in FIG. 3.

FIG. 3 shows graphs showing changes in the expression levels of each of the PSC-specific markers (OCT4, SOX2, and NANOG), the NCC-specific markers (p75NTR and PAX3), and the CEC-specific markers (ATP1A1, COL8A1, and AQP1), during a differentiation process from iPSCs to CECs via NCCs according to an aspect, wherein iPSC indicates induced pluripotent stem cells, NCC indicates iPSC-induced neural crest cells, D2 indicates cells on Day 2 of NCC to CEC induction, D4 indicates cells on Day 4 of NCC to CEC induction, D6 indicates cells on Day 6 of NCC to CEC induction, D8 indicates cells on Day 8 of NCC to CEC induction, and D10 indicates cells on Day 10 of NCC to CEC induction.

As shown in FIG. 3, it was confirmed that the expression of PSC-specific marker-associated genes was significantly high at the iPSC stage, but was hardly observed at other stages, and that NCC-specific marker-associated genes p75NTR and PAX3 were highly expressed at the NCC stage and CEC-specific marker-associated genes were most highly expressed on Days 4 to 6 of differentiation into CECs (p<0.05).

These results indicate the differentiation into CECs has been successfully achieved.

### Example 3. Confirmation of stability of CECs differentiated from iPSCs

To determine whether the CECs differentiated from the iPSCs of Example 1 maintained the characteristics of the CECs even after freezing and thawing, the expression of the CEC-specific markers (ATP1A1, COL8A1, and AQP1) was measured by qRT-PCR before and after thawing of the CECs differentiated from the iPSCs.

More specifically, the results measured from the iPSCs and CECs derived from iPSC-derived NCCs are shown in FIG. 4.

FIG. 4 shows graphs determining whether CEC-specific markers (ATP1A1, COL8A1, and AQP1) are expressed before and after freezing and thawing of CECs, wherein the CECs are differentiated according to an aspect and undergo freezing and thawing; iPSC indicates induced pluripotent stem cells, D4 indicates cells on Day 4 of induction of iPSC-derived NCC to CEC, and D6 indicates cells on Day 6 of induction of iPSC-induced NCC to CEC.

As shown in FIG. 4, when comparing the cells before and after freezing and thawing, D4 and D6 showed no significant difference in the expression of ATP1A1, COL8A1, and AQP1.

These results suggest that, even before and after freezing and thawing, the CECs according to an aspect maintain their stability well.

Furthermore, to determine the reversibility of the iPSC-derived CECs to iPSCs, the CECs were cultured in Stem MACS iPS-Brew XF medium. After 6 days of culture in the iPS-Brew XF medium, the expression of pluripotent markers was measured in the iPSC-derived CECS, and the results are shown in FIG. 5.

FIG. 5 shows graphs measuring the expression of pluripotent markers at 6 days after culturing the iPSC-derived CECs according to an aspect in the iPS-Brew XF medium.

As shown in FIG. 5, it was confirmed that the expression of pluripotent genes NANOG and OCT4 in the iPSC-derived CECs did not increase despite changing to iPSC culture medium.

Additionally, to determine whether the CECs differentiated according to an aspect exhibit oncogenicity, the iPSC-derived CECs (1X10⁶ cell/150 µL) were injected into immuno-suppressed mice. 2 months later, the mice were examined for teratoma formation. Then, the results are shown in FIG. 6.

FIG. 6 is an image of a mouse injected with the CECs differentiated according to an aspect and examined 2 months later for the formation of teratomas in the mouse.

As shown in FIG. 6, it was confirmed that the iPSC-derived CECs according to an aspect did not form any teratoma in vivo.

### Example 4. Confirmation of effects of transplantation of CECs differentiated from iPSCs

The CECs differentiated from the iPSCs of Example 1 were transplanted into a corneal endothelial dysfunction (CED) rabbit model. As the CED rabbit model, New Zealand white rabbits with artificially peeled Descemet's membrane in a circular pattern were used, and divided into a group transplanted with CECs differentiated from iPSCs (Group 1) and a control group not transplanted with the CECs (Group 2). These two groups were observed for 3 weeks, and the changes in the corneal opacity and corneal thickness were observed. Then, the results are shown in FIGS. 7 and 8.

The corneal opacity was evaluated on a scale of 0 to 4, and the criteria for determining the corneal opacity are shown in Table 1.

**[Table 1]**

| Score | Degree of corneal opacity |
|---|---|
| 0 | None |
| 1 | Mild opacity, clearly visible iris texture |
| 2 | Moderate opacity, unclear iris texture |
| 3 | Severe clouding, faded pupil |
| 4 | Severe clouding, invisible pupil |

FIG. 7A shows images determining whether the corneal transparency is improved after transplantation of CECs differentiated from iPSCs into the rabbit model of CED, compared to the cornea of the untransplanted CEB model, and FIG. 7B is a graph showing the changes in the corneal opacity over time in Group 1 and Group 2.

As shown in FIGS. 7A and 7B, it was confirmed that Group 1 had significant improvement in the corneal clouding phenomenon compared to Group 2. In addition, it was confirmed that, in Group 1 transplanted with the CECs, the corneal opacity improved proportionally over time after the CEC transplantation.

FIG. 8 shows images confirming the changes in the corneal thickness by H&E staining after transplanting the CECs differentiated from iPSCs into the CED rabbit, compared to the cornea of an untransplanted CED model; Scale bars: 200 µm.

As shown in FIG. 8, it was confirmed that the cornea of Group 1 transplanted with the CECs became thinned to a level similar to the corneal thickness of the normal control group. Meanwhile, it was confirmed that, in Group 2 not transplanted with the CECs, the cornea was still thick.

Additionally, the corneal tissue in Group 1 transplanted with the CECs was analyzed by immunohistochemical analysis at 3 weeks after the transplantation, and the results are shown in FIG. 9.

FIG. 9 shows images analyzing the corneal tissue in Group 1 transplanted with the CECs at 3 weeks after the transplantation, compared to the corneal tissue of a group not transplanted with the CECs.

As shown in FIG. 9, it was confirmed that the cells in Group 1 were derived from the transplanted CECs rather than the cells of the CED rabbit model itself. In addition, compared to untransplanted Group 2, the cells positive to ZO-1 and ATP1A1 increased in the cornea of Group 1 transplanted with the iPSC-derived CECs.

These results indicate that the CECs differentiated in vitro according to an aspect that can not only remain in the rabbit cornea for 3 weeks after the transplantation, but also exhibit an effective therapeutic effect while maintaining the histological characteristics.

### Example 5. Confirmation of effects of vitronectin (VTN) or fasudil (F) on efficient differentiation of stem cells into CECs

### 5.1 Conformation of effect by vitronectin

To determine the efficiency of differentiation induction into CECs using FNC mixture or vitronectin as a coating material for cell adhesion, the differentiation was induced according to the same protocol as in Example 1, except that a culture vessel coated with vitronectin was used instead of the culture vessel coated with the FNC mixture during the process of inducing differentiation of NCCs into CECs in Example 1. In addition, the expression levels of the PSC-specific markers (NANOG, SOX2, and OCT4) and the CEC-specific markers (ATP1A1, COL8A1, and AQP1) were measured by the method described in Reference Example 1, and the results are shown in FIG. 10.

FIG. 10 shows graphs measuring and comparing the changes in the expression levels of the PSC-specific markers (NANOG, SOX2, and OCT4) and the CEC-specific markers(ATP1A1, COL8A1, and AQP1) during a process of differentiating the stem cells into CECs via NCCs according to an aspect, wherein iPSC indicates induced pluripotent stem cells, D2 indicates cells on Day 2 of induction of iPSC-derived NCCs to CECs, D4 indicates cells on Day 4 of induction of iPSC-derived NCCs to CECs, and D6 indicates cells on Day 6 of induction of iPSC-derived NCCs to CECs.

As shown in FIG. 10, it was confirmed that the expression of the PSC-specific marker SOX2 was significantly high on Days 2, 4, and 6 of induction to CECs, when cultured in the culture vessel modified with the FNC mixture compared to the culture vessel modified with the vitronectin (p<0.05). In addition, it was confirmed that the expression of the CEC-specific markers COL8A1 and AQP1 was significantly high on Day 6 of induction into CECs, when cultured in the culture vessel modified with the vitronectin compared to the culture vessel modified with the FNC mixture (p<0.05).

These results indicate that the use of the culture vessel modified with the vitronectin during the process of iPSC to CEC differentiation reduces the pluripotency of the stem cells and increases the efficiency of differentiation into CECs, compared to use of the culture vessel modified with the FNC mixture.

### 5.2 Conformation of effect by fasudil and vitronectin

To confirm the effect of using F, differentiation from iPSCs into CECs was induced according to the same protocol as in Example 1, except that fasudil was used as the ROCK inhibitor and vitronectin was used as the coating material during a process of inducing differentiation from NCCs into CECs. In addition, compared to use of Y27632 as the ROCK inhibitor or use of FNC mixture as the coating material during a process of differentiating the NCCs into CECs, the effect of a combination of the coating material and the ROCK inhibitor on the induction of differentiation was confirmed.

First, whether a combination of the coating material (vitronectin or FNC mixture) and ROCK inhibitor (F or Y27632) effectively induced the differentiation of iPSCs into CECs was confirmed by whether the characteristic shape of the CECs was well maintained, and the results are shown in FIG. 11.

FIG. 11 shows images confirming the morphology of each cell among the cells induced to differentiate from iPSCs to CECs through different combinations of the coating material and the ROCK inhibitor, wherein VTN-Y indicates a combination of VTN and Y27632, VTN-F indicates a combination of vitronectin and fasudil, FNC-Y indicates a combination of FNC mixture and Y27632, and FNC-F indicates a combination of FNC mixture and fasudil.

As shown in FIG. 11, it was confirmed that VTN-Y, VTN-F, FNC-Y, and FNC-F all maintained the hexagonality, a characteristic shape of human-derived CECs, and all had a differentiation rate of 95 % or more.

In addition, the expression levels of the CEC-specific markers ZO-1, NCAM, SLC4A11, N-Cadherin, COL8A1, AQP1, and/or ATP1A1 were measured according to Reference Examples 2 and 3, and the results are shown in FIGS. 12A to 12C.

Additionally, the viability and cell proliferation levels of each combination was confirmed by CCK-8 assay, and the results are shown in FIG. 12D (*p < 0.05; N=3).

FIG. 12A shows images measuring the expression levels of the CEC-specific markers ZO-1, SLC4A11, N-Cadherin, and ATP1A1 in response to a combination of the coating material and the ROCK inhibitor during a process of iPSC to CEC induction according to an aspect; FIG. 12B is shows images measuring the expression levels of the CEC-specific markers ZO-1, NCAM, SLC4A11, N-Cadherin, and ATP1A1 in response to a combination of the coating material and the ROCK inhibitor during a process of iPSC to CEC induction according to an aspect; FIG. 12C shows graphs measuring the expression levels of the CEC-specific markers ATP1A1, COL8A1, and AQP1 in response to a combination of the coating material and the ROCK inhibitor during a process of iPSC to CEC induction according to an aspect, wherein iPSC indicates induced pluripotent stem cell, D2 indicates cells on Day 2 of induction of iPSC-derived NCCs to CECs, D4 indicates cells on Day 4 of induction of iPSC-derived NCCs to CECs, and D6 indicates cells on Day 6 of induction of iPSC-derived NCCs to CECs; and FIG. 12D is a graph showing the cell viability and cell proliferation levels in response to a combination of the coating material and the ROCK inhibitor during a process of iPSC to CEC induction according to an aspect, wherein VTN-Y indicates a combination of vitronectin and Y27632, VTN-F indicates a combination of vitronectin and fasudil, FNC-Y indicates a combination of FNC mixture and Y27632, and FNC-F indicates a combination of FNC mixture and fasudil.

As shown in FIG. 12, the CEC-specific marker was most highly expressed in response to the VTN-F indicating the combination of vitronectin and fasudil, confirming that a combination of fasudil and vitronectin is the optimal condition for the differentiation and growth of CECs.

Specifically, as shown in FIG. 12A, it was confirmed that the expression of the CEC-specific markers ZO-1 and ATP1A1 was increased the most in response to the combination using the vitronectin instead of the FNC mixture, which is an expensive coating material. In addition, as shown in FIG. 12B, it was confirmed that the expression of ATP1A1, which is responsible for pump function, was increased the most among the CEC-specific markers in response to the combination using the vitronectin instead of the FNC mixture, which is an expensive coating material.

In addition, it was confirmed that the cell viability and proliferation levels were increased in the fasudil-treated group compared to the Y27632-treated group.

These results indicate that replacing the FNC mixture and the Y-27632 with the vitronectin and the fasudil may increase the efficiency of stem cell to CEC differentiation and ultimately benefit clinical trials.

### Example 6. Promotion of stem cell to CEC differentiation in response to combination of vitronectin and fasudil

Differentiation of stem cells into CECs was induced according to the same protocol as in Example 1, except that, among the medium components used for the NCC to CEC differentiation, Y-27632 was changed to fasudil and the coating material was changed from FNC mixture to vitronectin. Here, to determine whether the iPSC-derived CECs are effectively transplanted into the cornea when transplanted into a CED animal model, stem cells transfected with a green fluorescent protein (ZsGreen) was used as the stem cells. Then, the induced CECs were transplanted into CED rabbit models which were then divided into a group transplanted with CECs differentiated from the iPSCs and a group not receiving the CECs. Afterwards, the degree of corneal opacity was examined for 3 weeks, and the expression patterns of ZO-1 and ATP1A1 were observed in the transplanted corneal tissue. As the CED rabbit models, New Zealand white rabbits with artificially peeled Descemet's membrane in a circular pattern were used, and the results are shown in FIG. 13.

FIG. 13A shows images confirming changes in the corneal opacity in the CED rabbit model after transplanting CECs induced in response to a combination of vitronectin and fasudil (i.e., iPSC-CECs(VTN with fasudil)) to the cornea of the CED rabbit model; FIG. 13B shows images confirming the CECs expressing a green fluorescent protein observed in the cornea transplanted with iPSC(ZsGreen)-derived CECs(i.e., iPSC(ZsGreen)-CECs(VTN with fasudil)); FIG. 13C shows images confirming positive cells expressing human CEC-specific markers ZO-1 and ATP1A1 in the corneal tissue transplanted with the iPSC-CECs(VTN with fasudil); and FIG. 13D shows images confirming changes in the corneal opacity observed for 20 weeks after transplanting the iPSC-CECs(VTN with fasudil)) into the cornea of the CED rabbit model.

As shown in FIG. 13, it was confirmed that the corneal opacity was significantly ameliorated in the rabbit models transplanted with either the iPSC-CEC(VTN with fasudil) or the iPSC(ZsGreen)-CEC (VTN with fasudil) compared to the untransplanted control group. In addition, it was confirmed that the ZO-1 and ATP1A1 positive cells were observed in the corneal tissue transplanted with the iPSC-CEC(VTN with fasudil).

These results suggest that CECs differentiated from iPSCs can survive after transplantation and effectively treat corneal endothelial dysfunction.

## Claims

1. A method of differentiating stem cells into corneal endothelial cells, the method comprising culturing stem cells in a culture vessel coated with a modified extracellular matrix (ECM) in the presence of a composition containing a Rho-associated protein kinase (ROCK) inhibitor.

2. The method of claim 1, wherein the ROCK inhibitor is at least one selected from the group consisting of fasudil, N-(4-pyridinyl)-4β-[(R)-1-aminoethyl]cyclohexane-1αcarbamide (Y-27632), (2S)-2-methyl-1-[(4-methyl-5-isoquinolinyl)sulfonyl]hexahydro-1H-1,4-diazepine (H-1152), 4β-[(1R)-1-aminoethyl]-N-(4-pyridyl)benzene-1zencarboamide (Wf-536), N-(1H-pyrrolo[2,3-b]pyridin-4-yl)-4PER(R)-1-aminoethyl]cyclohexane-1rohecarboamide (Y-30141), N-(3-{[2-(4-amino-1,2,5-oxadiazol-3-yl)-1-ethyl-1H-imidazo[4,5-c]pyridin-6-yl]oxy}phenyl)-4-{[2-(4-morpholinyl)ethyl]-oxy}benzamide (GSK269962A), and N-(6-fluoro-1H-indazol-5-yl)-6-methyl-2-oxo-4-[4-(trifluoromethyl)phenyl]-3,4-dihydro-1H-pyridine-5-carboxamide (GSK429286A).

3. The method of claim 1, wherein the ECM contains at least one selected from the group consisting of vitronectin, collagen, f ibronectin, laminin, gelatin, matrigel, hyaluronic acid, polylysine, and heparan sulfate proteoglycan.

4. The method of claim 1, wherein the stem cells are embryonic stem cells (ESCs), adult stem cells, or human induced pluripotent stem cells (iPSCs).

5. The method of claim 1, wherein the method comprises:
differentiating the stem cells into neural crest cells (NCCs) by culturing the stem cells in a culture vessel to which a first medium is added; and
differentiating the NCCs into corneal endothelial cells by culturing the NCCs in a culture vessel to which a second medium is added.

6. The method of claim 5, wherein the differentiating of the stem cells into NCCs comprises culturing the stem cells for 1 to 10 days in the culture vessel to which the first medium is added.

7. The method of claim 5, wherein the differentiating of the NCCs into corneal endothelial cells comprises culturing the NCCs for 2 to 15 days in the culture vessel to which the second medium is added.

8. The method of claim 1, wherein the composition contains polyvinyl alcohol (PVA).

9. The method of claim 1, wherein the composition is fetal bovine serum (FBS)-free.

10. A kit for inducing differentiation of stem cells into corneal endothelial cells, the kit comprising:
a Rho-associated protein kinase (ROCK) inhibitor; and
a culture vessel coated with a modified extracellular matrix (ECM).

11. The kit of claim 10, wherein the ROCK inhibitor is at least one selected from the group consisting of fasudil, N-(4-pyridinyl)-4β-[(R)-1-aminoethyl]cyclohexane-1αcarbamide (Y-27632), (2S)-2-methyl-1-[(4-methyl-5-isoquinolinyl)sulfonyl]hexahydro-1H-1,4-diazepine (H-1152), 4β-[(1R)-1-aminoethyl]-N-(4-pyridyl)benzene-1zencarbamide (Wf-536), N-(1H-pyrrolo[2,3-b]pyridin-4-yl)-4PER(R)-1-aminoethyl]cyclohexane-1rohecarbamide (Y-30141), N-(3-{[2-(4-amino-1,2,5-oxadiazol-3-yl)-1-ethyl-1 H-im idazo[4,5-c]pyridin-6-yl]oxy}phenyl)-4-{[2-(4-morpholinyl)ethyl]-oxy}benzamide (GSK269962A), and N-(6-fluoro-1H-indazol-5-yl)-6-methyl-2-oxo-4-[4-(trifluoromethyl)phenyl]-3,4-dihydro-1H-pyridine-5-carboxamide (GSK429286A).

12. The kit of claim 10, wherein the ECM contains at least one selected from the group consisting of vitronectin, collagen, fibronectin, laminin, gelatin, matrigel, hyaluronic acid, polylysine, and heparan sulfate proteoglycan.

13. The kit of claim 10, wherein the composition contains polyvinyl alcohol (PVA).

14. The kit of claim 10, wherein the composition is fetal bovine serum (FBS)-free.

15. The kit of claim 10, wherein the stem cells are embryonic stem cells (ESCs), adult stem cells, or human induced pluripotent stem cells (iPSCs).
